# EUROPEAN PATENT APPLICATION

(11) **EP 4 710 926 A1**
(43) Date of publication of application: **18.03.2026**
(21) Application number: 24807134.2
(22) Date of filing: 10.05.2024
(51) Int. Cl.: A61K 31/593, A61K 9/06, A61K 9/08, A61K 9/12, A61K 31/573, A61K 47/14, A61K 47/34, A61K 47/44, A61P 17/06, A61P 43/00

(54) **TOPICAL COMPOSITION**

(30) Priority: 12.05.2023 JP 2023079431
(71) Applicant: Api Co., Ltd., Gifu 500-8558 (JP)
(72) Inventor: UMEMOTO, Shota, Gifu-shi, Gifu 500-8558 (JP); FUKUCHI, Tomoya, Gifu-shi, Gifu 500-8558 (JP)
(74) Representative: Hoffmann Eitle
(86) International application number: PCT/JP2024/017356
(87) International publication number: WO 2024/237182

(57) **Abstract**

The present invention provides a technique for further improving the stability of vitamin D3 in a topical composition comprising vitamin D3, even when the amount of water present in the topical composition is increased. Provided is a topical composition comprising vitamin D3, a nonionic surfactant A, which is a polyoxyalkylene hydrogenated castor oil having an average number of moles of alkylene oxide added of 55 or less, and a nonionic surfactant B, which is a nonionic surfactant other than polyoxyalkylene hydrogenated castor oils.

## Description

### Technical Field

The present invention relates to a topical composition and the like.

### Background Art

Psoriasis is a skin disease whose main symptoms include skin keratinization, scale formation, skin thickening, reddish rash, and the like, and there are several hundred thousand patients in Japan. It is known that vitamin D3 is used as a topical agent for psoriasis skin symptoms (see PTL 1).

### Citation List

### Patent Literature

PTL 1: JP2021-518409A

### Summary of Invention

### Technical Problem

Although vitamin D3 has excellent therapeutic efficacy, its stability decreases upon dissolution in water. As the water content increases, the issue of stability becomes increasingly significant.

Accordingly, an object of the present invention is to provide a technique for further improving the stability of vitamin D3 in a topical composition comprising vitamin D3, even when the amount of water present in the topical composition is increased.

### Solution to Problem

In view of the object mentioned above, the present inventors conducted extensive research and then found that the above object can be achieved by a topical composition comprising vitamin D3, a nonionic surfactant A, which is a polyoxyalkylene hydrogenated castor oil, and a nonionic surfactant B, which is a nonionic surfactant other than polyoxyalkylene hydrogenated castor oils. Based on such findings, the inventors conducted further research. The present invention has thus been completed. Specifically, the present invention encompasses the following aspects.

### Item 1.

A topical composition comprising vitamin D3, a nonionic surfactant A, which is a polyoxyalkylene hydrogenated castor oil having an average number of moles of alkylene oxide added of 55 or less, and a nonionic surfactant B, which is a nonionic surfactant other than polyoxyalkylene hydrogenated castor oils.

### Item 2.

The topical composition according to Item 1, wherein the nonionic surfactant B is at least one selected from the group consisting of polyoxyalkylene alkyl ethers, polyoxyalkylene glycols, polyoxyalkylene fatty acid esters, polyoxyalkylene sorbitol fatty acid esters, and polyglycerol fatty acid esters.

### Item 3.

The topical composition according to Item 1 or 2, wherein the nonionic surfactant B has an HLB value of 6 or more.

### Item 4.

The topical composition according to any one of Items 1 to 3, wherein the total content of the nonionic surfactant A and the nonionic surfactant B in the topical composition is 0.5 mass% or more and 10 mass% or less.

### Item 5.

The topical composition according to any one of Items 1 to 4, wherein the content of the nonionic surfactant A in the total of the nonionic surfactant A and the nonionic surfactant B is 10 mass% or more and 99.9 mass% or less.

### Item 6.

The topical composition according to any one of Items 1 to 5, which comprises an ester oil.

### Item 7.

The topical composition according to claim 6, wherein the ester oil comprises at least glyceryl triisooctanoate.

### Item 8.

The topical composition according to any one of Items 1 to 7, wherein
the nonionic surfactant B is at least one selected from the group consisting of polyoxyalkylene alkyl ethers, polyoxyalkylene glycols, polyoxyalkylene fatty acid esters, polyoxyalkylene sorbitol fatty acid esters, and polyglycerol fatty acid esters,
the nonionic surfactant B has an HLB value of 6 or more,
the total content of the nonionic surfactant A and the nonionic surfactant B in the topical composition is 1 mass% or more and 10 mass% or less,
the content of the nonionic surfactant A in the total of the nonionic surfactant A and the nonionic surfactant B is 10 mass% or more and 99.9 mass% or less, and
the topical composition comprises an ester oil.

### Item 9.

The topical composition according to any one of Items 1 to 8, wherein the vitamin D3 is at least one selected from the group consisting of calcipotriol, maxacalcitol, alfacalcidol, falecalcitriol, tacalcitol, and calcitriol.

### Item 10.

The topical composition according to any one of Items 1 to 9, wherein the nonionic surfactant B is at least one selected from polyoxyethylene polyoxypropylene cetyl ether, polyoxyethylene lauryl ether, polyoxyethylene polyoxypropylene glycol, polyethylene glycol monostearate, polyoxyethylene sorbitol tetraoleate, and polyglyceryl monolaurate.

### Item 11.

The topical composition according to any one of Items 1 to 10, which further comprises water.

### Item 12.

The topical composition according to Item 11, wherein the content of water in the topical composition is 40 mass% or more.

### Item 13.

The topical composition according to Item 11, wherein the content of water in the topical composition is 75 mass% or more.

### Item 14.

The topical composition according to any one of Items 1 to 13, wherein the content of the vitamin D3 in the topical composition is 0.0001 mass% or more and 0.2 mass% or less.

### Item 15.

The topical composition according to any one of Items 1 to 14, which further comprises an antioxidant.

### Item 16.

The topical composition according to any one of Items 1 to 15, wherein the dosage form is a gel, liquid, aerosol, mist, or foam.

### Item 17.

The topical composition according to any one of Items 1 to 16 for use in the prevention or amelioration of psoriasis.

### Advantageous Effects of Invention

The present invention can provide a topical composition that comprises vitamin D3, and that has further improved stability of vitamin D3, even when the amount of water present in the composition is increased.

### Brief Description of Drawings

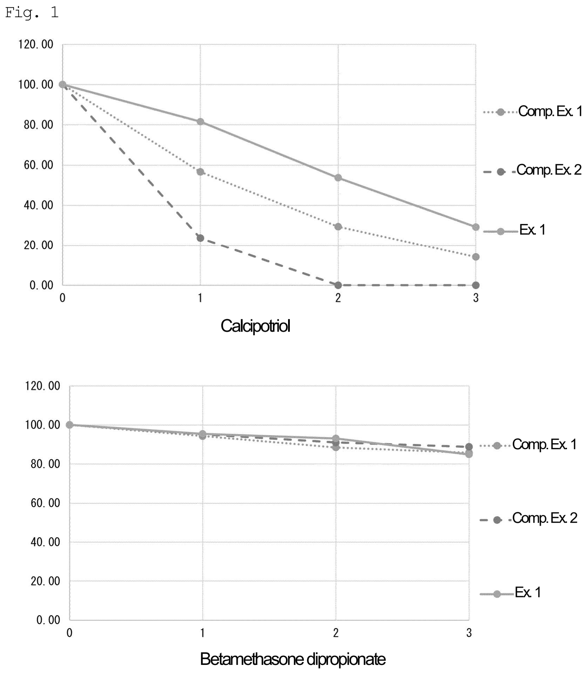

Fig. 1 shows the residual rate (vertical axis) of vitamin D3 (upper graph) or glucocorticoid (lower graph) when liquid compositions (Example 1, Comparative Example 1, and Comparative Example 2) were left to stand at 60°C. The horizontal axis represents the number of weeks elapsed since the start of standing at 60°C.

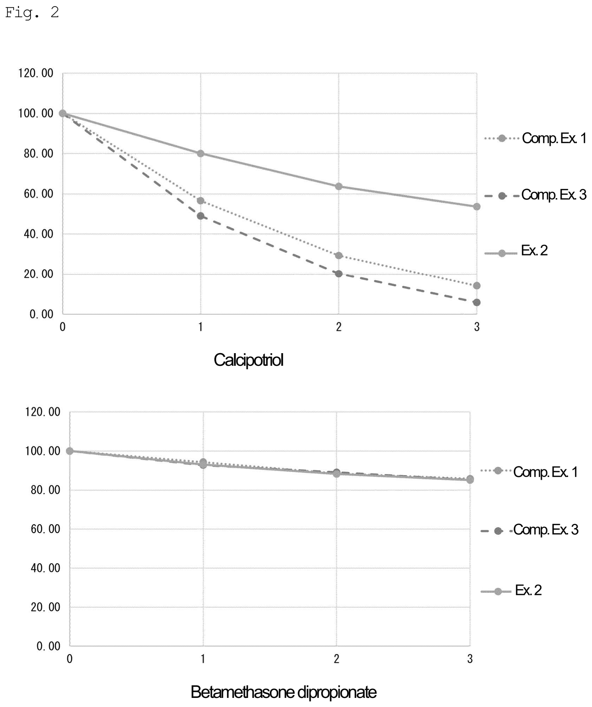

Fig. 2 shows the residual rate (vertical axis) of vitamin D3 (upper graph) or glucocorticoid (lower graph) when liquid compositions (Example 2, Comparative Example 1, and Comparative Example 3) were left to stand at 60°C. The horizontal axis represents the number of weeks elapsed since the start of standing at 60°C.

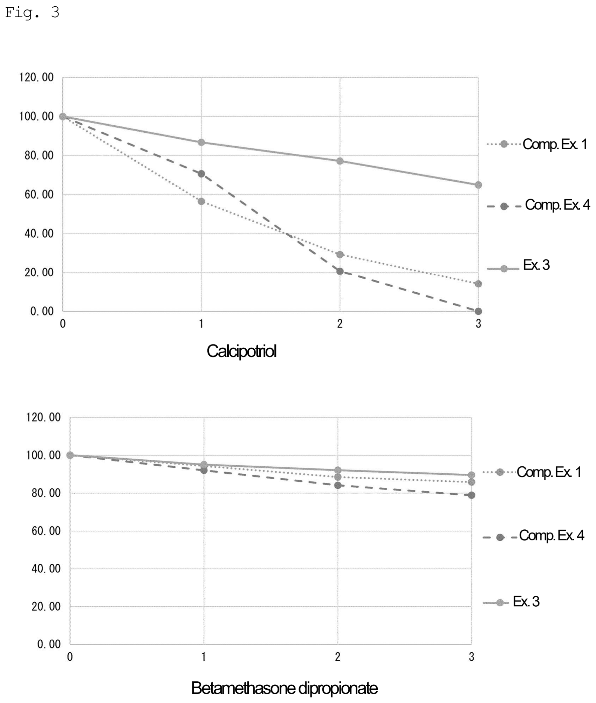

Fig. 3 shows the residual rate (vertical axis) of vitamin D3 (upper graph) or glucocorticoid (lower graph) when liquid compositions (Example 3, Comparative Example 1, and Comparative Example 4) were left to stand at 60°C. The horizontal axis represents the number of weeks elapsed since the start of standing at 60°C.

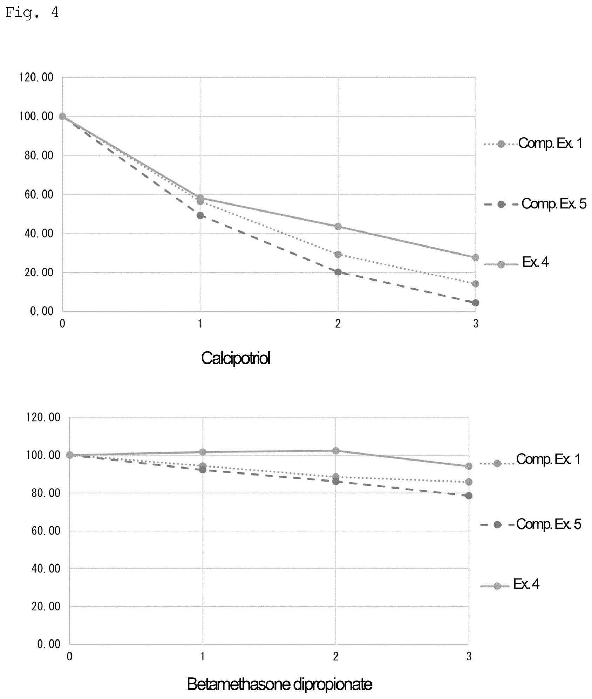

Fig. 4 shows the residual rate (vertical axis) of vitamin D3 (upper graph) or glucocorticoid (lower graph) when liquid compositions (Example 4, Comparative Example 1, and Comparative Example 5) were left to stand at 60°C. The horizontal axis represents the number of weeks elapsed since the start of standing at 60°C.

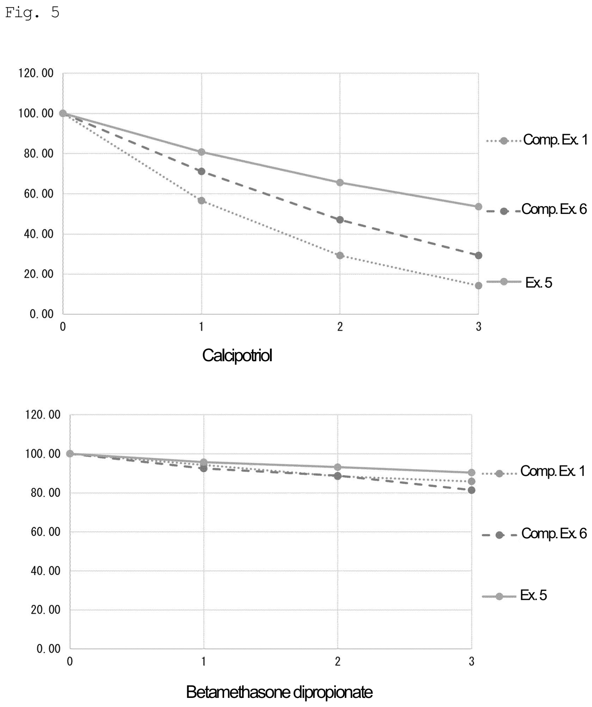

Fig. 5 shows the residual rate (vertical axis) of vitamin D3 (upper graph) or glucocorticoid (lower graph) when liquid compositions (Example 5, Comparative Example 1, and Comparative Example 6) were left to stand at 60°C. The horizontal axis represents the number of weeks elapsed since the start of standing at 60°C.

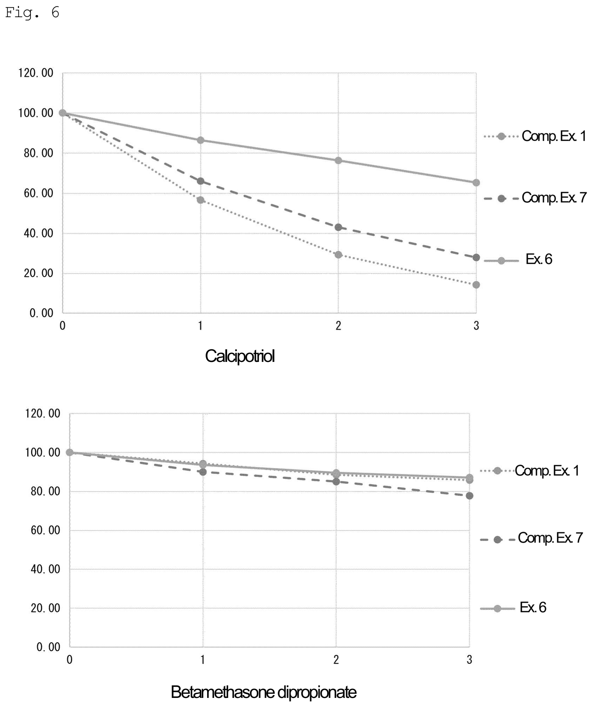

Fig. 6 shows the residual rate (vertical axis) of vitamin D3 (upper graph) or glucocorticoid (lower graph) when liquid compositions (Example 6, Comparative Example 1, and Comparative Example 7) were left to stand at 60°C. The horizontal axis represents the number of weeks elapsed since the start of standing at 60°C.

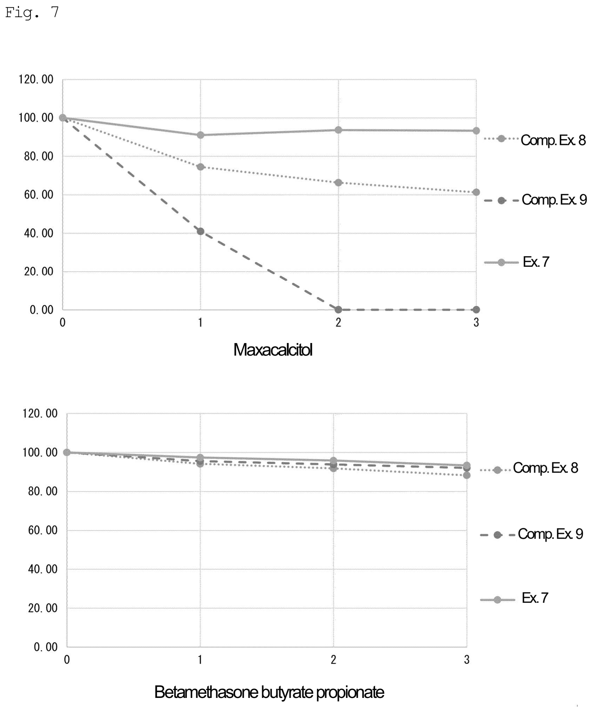

Fig. 7 shows the residual rate (vertical axis) of vitamin D3 (upper graph) or glucocorticoid (lower graph) when liquid compositions (Example 7, Comparative Example 8, and Comparative Example 9) were left to stand at 60°C. The horizontal axis represents the number of weeks elapsed since the start of standing at 60°C.

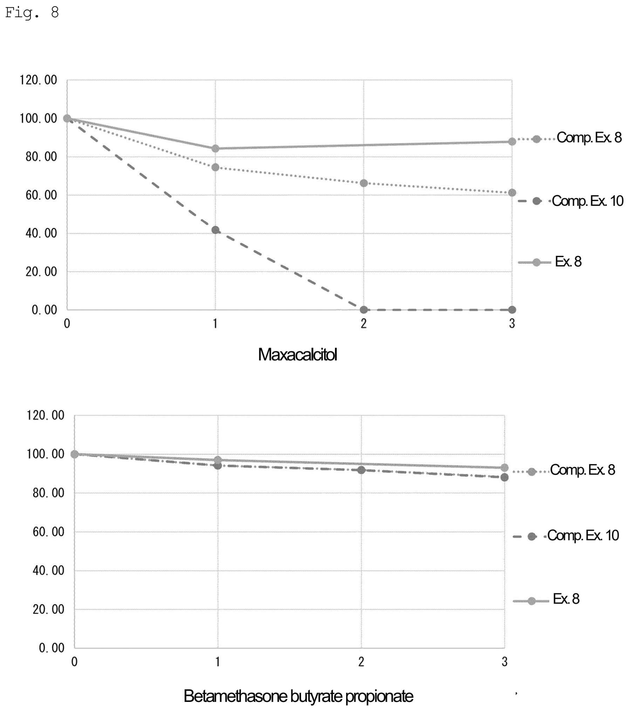

Fig. 8 shows the residual rate (vertical axis) of vitamin D3 (upper graph) or glucocorticoid (lower graph) when liquid compositions (Example 8, Comparative Example 8, and Comparative Example 10) were left to stand at 60°C. The horizontal axis represents the number of weeks elapsed since the start of standing at 60°C.

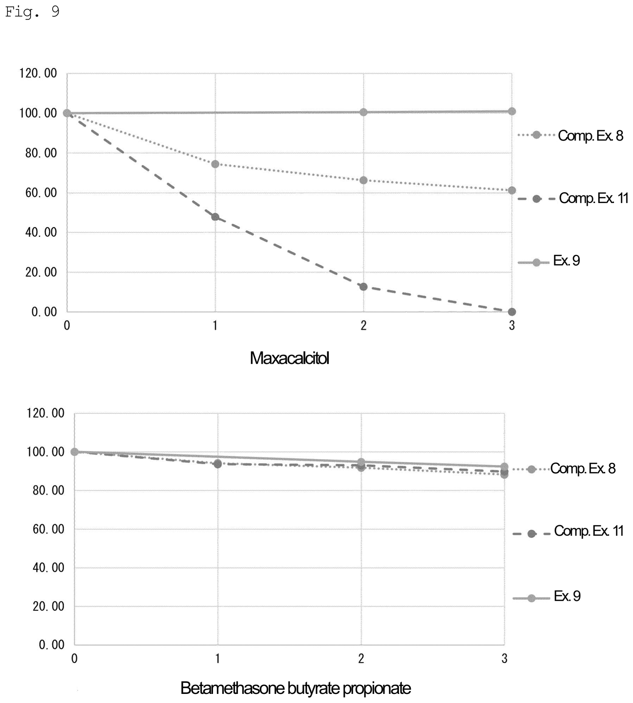

Fig. 9 shows the residual rate (vertical axis) of vitamin D3 (upper graph) or glucocorticoid (lower graph) when liquid compositions (Example 9, Comparative Example 8, and Comparative Example 11) were left to stand at 60°C. The horizontal axis represents the number of weeks elapsed since the start of standing at 60°C.

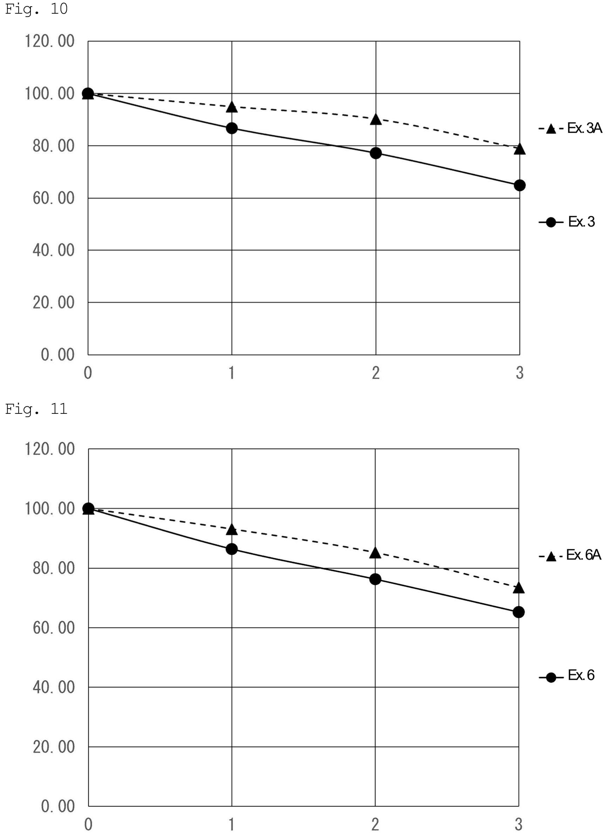

Fig. 10 shows the residual rate (vertical axis) of vitamin D3 when liquid compositions (Example 3 and Example 3A) were left to stand at 60°C. The horizontal axis represents the number of weeks elapsed since the start of standing at 60°C.
Fig. 11 shows the residual rate (vertical axis) of vitamin D3 when liquid compositions (Example 6 and Example 6A) were left to stand at 60°C. The horizontal axis represents the number of weeks elapsed since the start of standing at 60°C.

### Description of Embodiments

In the present specification, the terms "contain" and "comprise" encompass the concepts of containing, comprising, substantially consisting of, and consisting of.

In the present specification, the number of carbon atoms in "alkylene" is, for example, 2 to 4, preferably 2 to 3, and more preferably 2.

In one aspect, the present invention relates to a topical composition comprising vitamin D3, a nonionic surfactant A, which is a polyoxyalkylene hydrogenated castor oil having an average number of moles of alkylene oxide added of 55 or less, and a nonionic surfactant B, which is a nonionic surfactant other than polyoxyalkylene hydrogenated castor oils (also referred to in the present specification as "the topical composition of the present invention"). This composition is described below.

Vitamin D3 is not particularly limited as long as it is usable for active vitamin D3 preparations. Examples of vitamins D3 include calcipotriol (1α,24(OH)₂-22-ene-24-cyclopropyl-vitamin D3), maxacalcitol (22-oxa-1α,25-dihydroxyvitamin D3), alfacalcidol, falecalcitriol (1,25-dihydroxy-26,27-hexafluoro-vitamin D3), tacalcitol (1α,24(R)-dihydroxyvitamin D3), and calcitriol (1,25-dihydroxyvitamin D3). These components encompass free forms, salts, and solvates (for example, hydrates). Among these, from the viewpoint of the stability of the drug (in particular, vitamin D3), calcipotriol and maxacalcitol are preferred. In one aspect of the present invention, vitamin D3 is particularly preferably maxacalcitol. In another aspect of the present invention, vitamin D3 is calcipotriol.

Vitamins D3 may be used alone or in any combination of two or more.

The content of the vitamin D3 in the topical composition of the present invention is not particularly limited and may be, for example, set to 0.0001 mass% or more and 0.2 mass% or less. The content is preferably 0.0002 mass% or more and 0.1 mass% or less, preferably 0.0005 mass% or more and 0.05 mass% or less, preferably 0.001 mass% or more and 0.02 mass% or less, and preferably 0.0015 mass% or more and 0.01 mass% or less.

The polyoxyalkylene hydrogenated castor oil (nonionic surfactant A) is obtained by addition polymerization of an alkylene oxide with hydrogenated castor oil, and is not particularly limited as long as it has an average number of moles of alkylene oxide added of 55 or less. From the viewpoint of the stability of vitamin D3, the average number of moles of ethylene oxide added is further preferably 10 or more and 55 or less, still more preferably 20 or more and 55 or less, even more preferably 30 or more and 50 or less, and particularly preferably 35 or more and 45 or less. The polyoxyalkylene hydrogenated castor oil is particularly preferably a polyoxyethylene hydrogenated castor oil. Specific examples of polyoxyethylene hydrogenated castor oils include polyoxyethylene (5) hydrogenated castor oil (also referred to as "polyoxyethylene hydrogenated castor oil 5"), polyoxyethylene (10) hydrogenated castor oil (also referred to as "polyoxyethylene hydrogenated castor oil 10"), polyoxyethylene (20) hydrogenated castor oil (also referred to as "polyoxyethylene hydrogenated castor oil 20"), polyoxyethylene (30) hydrogenated castor oil (also referred to as "polyoxyethylene hydrogenated castor oil 30"), polyoxyethylene (40) hydrogenated castor oil (also referred to as "polyoxyethylene hydrogenated castor oil 40"), and polyoxyethylene (50) hydrogenated castor oil (also referred to as "polyoxyethylene hydrogenated castor oil 50").

The nonionic surfactants A may be used alone or in any combination of two or more.

The content of the nonionic surfactant A in the topical composition of the present invention is not particularly limited and is, for example, 0.1 mass% or more and 20 mass% or less. From the viewpoint of, for example, the stability of vitamin D3 and a reduction in the stickiness of the topical composition, the content is preferably 0.2 mass% or more and 15 mass% or less, more preferably 0.5 mass% or more and 10 mass% or less, still more preferably 1 mass% or more and 8 mass% or less, even more preferably 1.5 mass% or more and 6 mass% or less, and particularly preferably 2 mass% or more and 5 mass% or less.

The nonionic surfactant B is not particularly limited as long as it is a nonionic surfactant other than polyoxyalkylene hydrogenated castor oils. Examples of the nonionic surfactant B include polyoxyalkylene alkyl ethers, polyoxyalkylene glycols, polyoxyalkylene fatty acid esters, polyoxyalkylene sorbitol fatty acid esters, polyglycerol fatty acid esters, polyoxyalkylene glycerin fatty acid esters, and polyoxyalkylene sorbitan fatty acid esters.

Examples of polyoxyalkylene alkyl ethers include polyoxyethylene lauryl ether, polyoxyethylene cetyl ether, polyoxyethylene stearyl ether, polyoxyethylene oleyl ether, polyoxyethylene myristyl ether, polyoxyethylene palmityl ether, polyoxyethylene isostearyl ether, polyoxyethylene hexyldecyl ether, polyoxyethylene behenyl ether, polyoxyethylene linoleyl ether, polyoxyethylene capryl ether, polyoxyethylene dodecyl ether, polyoxyethylene tridecyl ether, polyoxyethylene isotridecyl ether, polyoxyethylene pentadecyl ether, polyoxyethylene cholestanol ether, polyoxyethylene octyldodecyl ether, and polyoxyethylene polyoxypropylene decyltetradecyl ether.

Examples of polyoxyalkylene glycols include polyoxyethylene polyoxypropylene glycol.

Examples of polyoxyalkylene fatty acid esters include polyethylene glycol monostearate, polyethylene glycol distearate, polyethylene glycol monooleate, polyethylene glycol dioleate, polyethylene glycol monoisostearate, and polyethylene glycol diisostearate.

Examples of polyoxyalkylene sorbitol fatty acid esters include polyoxyethylene sorbitol tetraoleate, polyoxyethylene sorbitol hexastearate, polyoxyethylene sorbitol monolaurate, and polyoxyethylene sorbitol beeswax.

Examples of polyglycerol fatty acid esters include diglyceryl monocaprylate, decaglyceryl monocaprylate, hexaglyceryl monocaprylate, tetraglyceryl monolaurate, hexaglyceryl monolaurate, decaglyceryl monolaurate, polyglyceryl monolaurate, decaglyceryl monomyristate, decaglyceryl monostearate, decaglyceryl monoisostearate, polyglyceryl monostearate, diglyceryl monooleate, hexaglyceryl monooleate, diglyceryl sesquioleate, polyglyceryl diisostearate, polyglyceryl distearate, diglyceryl triisostearate, and polyglyceryl tristearate.

Examples of polyoxyalkylene glycerin fatty acid esters include polyoxyethylene glycerin monostearate.

Examples of polyoxyalkylene sorbitan esters include polyoxyethylene sorbitan monostearate, polyoxyethylene sorbitan monolaurate, polyoxyethylene sorbitan tristearate, and polyoxyethylene sorbitan trioleate.

In addition to those listed above, examples of the nonionic surfactant B also include glycerin fatty acid esters, sorbitan fatty acid esters, and alkyl glyceryl ethers (ethers formed by the reaction of higher alcohols and glycerin).

The HLB (hydrophilic-lipophilic balance) value of the nonionic surfactant is preferably 6 or more, more preferably 6.5 or more, still more preferably 7 or more, even more preferably 7 to 19, and particularly preferably 7 to 18, from the viewpoint of more effectively improving the stability of the drug (in particular, vitamin D3).

The nonionic surfactants B may be used alone or in any combination of two or more.

The content of the nonionic surfactant B in the topical composition of the present invention is not particularly limited and is, for example, 0.002 mass% or more and 10 mass% or less. From the viewpoint of, for example, the stability of vitamin D3 and a reduction in the stickiness of the topical composition, the content is preferably 0.005 mass% or more and 10 mass% or less, more preferably 0.01 mass% or more and 10 mass% or less, still more preferably 0.02 mass% or more and 10 mass% or less, even more preferably 0.05 mass% or more and 8 mass% or less, still even more preferably 0.1 mass% or more and 6 mass% or less, particularly preferably 0.2 mass% or more and 4 mass% or less, and more particularly preferably 0.5 mass% or more and 3 mass% or less.

The total content of the nonionic surfactant A and the nonionic surfactant B in the topical composition of the present invention is not particularly limited and is, for example, 0.1 mass% or more and 20 mass% or less. From the viewpoint of, for example, the stability of vitamin D3 and a reduction in the stickiness of the topical composition, the total content is preferably 0.5 mass% or more and 15 mass% or less, more preferably 1 mass% or more and 12 mass% or less, still more preferably 2 mass% or more and 10 mass% or less, even more preferably 3 mass% or more and 8 mass% or less, and particularly preferably 4 mass% or more and 6 mass% or less.

The content of the nonionic surfactant A in the total of the nonionic surfactant A and the nonionic surfactant B is not particularly limited and is, for example, 10 mass% or more and 99.9 mass% or less. From the viewpoint of, for example, the stability of vitamin D3 and a reduction in the stickiness of the topical composition, the content is preferably 20 mass% or more and 99.5 mass% or less, more preferably 30 mass% or more and 99 mass% or less, still more preferably 40 mass% or more and 95 mass% or less, even more preferably 45 mass% or more and 92 mass% or less, and particularly preferably 45 mass% or more and 90 mass% or less.

The topical composition of the present invention preferably comprises an ester oil. The presence of an ester oil can further improve the stability of vitamin D3. The ester oil is an oily component composed of an ester of a fatty acid and an alcohol, and is not particularly limited as long as it is liquid at an ordinary temperature (20°C).

Specific examples of ester oils include those listed below.

Ester oils of a dibasic acid, such as dioctyl succinate, diisopropyl adipate, diisobutyl adipate, dioctyl adipate, diethyl sebacate, diisopropyl sebacate, and dioctyl sebacate.

Ester oils having a hydroxyl group, such as lauryl lactate, myristyl lactate, cetyl lactate, octyldodecyl lactate, trioctyl citrate, triisocetyl citrate, trioctyldodecyl citrate, and diisostearyl malate.

Ester oils of a straight-chain fatty acid with a lower alcohol, such as isopropyl myristate, butyl myristate, isopropyl palmitate, ethyl stearate, butyl stearate, ethyl oleate, ethyl linoleate, and isopropyl linoleate.

Ester oils of a straight-chain fatty acid with a straight-chain higher alcohol, such as cetyl caprate, hexyl laurate, decyl myristate, myristyl myristate, cetyl myristate, cetyl palmitate, stearyl stearate, decyl oleate, and oleyl oleate.

Ester oils of a straight-chain fatty acid with an alcohol having a branched chain, such as isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, and octyldodecyl ricinoleate.

Ester oils of a fatty acid having a branched chain with a lower alcohol, such as ethyl isostearate and isopropyl isostearate.

Ester oils of a fatty acid having a branched chain with a straight-chain higher alcohol, such as cetyl 2-ethylhexanoate, cetostearyl 2-ethylhexanoate, stearyl 2-ethylhexanoate, hexyl isostearate, and 2-hexyldecyl isostearate.

Ester oils of a fatty acid with a polyhydric alcohol, such as ethylene glycol dioctanoate, ethylene glycol dioleate, propylene glycol dicaprylate, propylene glycol di(caprylate/caprate), propylene glycol dicaprate, dipropylene glycol dioleate, neopentyl glycol dicaprate, neopentyl glycol dioctanoate, glyceryl tricaprylate, glyceryl tri-2-ethylhexanoate, glyceryl triisooctanoate, glyceryl tri(caprylate/caprate), glyceryl triisopalmitate, glyceryl triisostearate, trimethylolpropane triisostearate, pentaerythritol tetra-2-ethylhexanoate, pentaerythritol tetra-isostearate, and pentaerythrityl tetra-2-ethylhexanoate.

Ester oils of a fatty acid having a branched chain with an alcohol having a branched chain, such as octyldodecyl neopentanoate, isocetyl octanoate, isostearyl octanoate, 2-ethylhexyl isononanoate, hexyldecyl dimethyloctanoate, octyldodecyl dimethyloctanoate, 2-ethylhexyl isopalmitate, isocetyl isostearate, isostearyl isostearate, and octyldodecyl isostearate.

Among the specific examples listed above, the topical composition of the present invention can preferably comprise an ester oil of a dibasic acid, from the viewpoint of, for example, the stability of vitamin D3. From the same viewpoint, the ester oil of a dibasic acid to be contained is more preferably a diester (preferably a symmetrical diester) of a dicarboxylic acid (preferably a linear dicarboxylic acid) whose main chain has 4 to 8 (preferably 5 to 7) carbon atoms with an alkyl alcohol (preferably having 2 to 10, more preferably 3 to 10, and even more preferably 3 to 6 carbon atoms).

In one aspect of the present invention, the ester oil can comprise at least glyceryl triisooctanoate.

The ester oils may be used alone or in any combination of two or more.

In cases in which the topical composition of the present invention comprises an ester oil, the content of the ester oil in the topical composition of the present invention is not particularly limited and is, for example, more than 0 mass% and 10 mass% or less. From the viewpoint of, for example, the stability of vitamin D3, the content is preferably 0.005 mass% or more and 10 mass% or less, more preferably 0.05 mass% or more and 10 mass% or less, still more preferably 0.1 mass% or more and 8 mass% or less, even more preferably 0.2 mass% or more and 6 mass% or less, particularly preferably 0.5 mass% or more and 4 mass% or less, and more particularly preferably 1 mass% or more and 2 mass% or less.

The topical composition of the present invention preferably comprises a glucocorticoid. The present invention can provide a topical composition in which vitamin D3 is more stable and the glucocorticoid is also stable.

The glucocorticoid is not particularly limited as long as it is usable for steroid preparations. Examples of glucocorticoids include cortisones, such as cortisone, hydrocortisone, cortisol, and corticosterone; prednisolones, such as prednisolone and methylprednisolone; and dexamethasone, betamethasone, and derivatives thereof. Examples of derivatives include esters, such as phosphate ester, valerate ester, butyrate ester, and propionate ester; these esters may be single acid esters or mixed acid esters. Among these, betamethasone or esters thereof are preferred. Among the esters, betamethasone dipropionate, betamethasone butyrate propionate, and the like are preferred.

The glucocorticoids may be used alone or in any combination of two or more.

In cases in which the topical composition of the present invention comprises a glucocorticoid, the content of glucocorticoid in the topical composition of the present invention is not particularly limited and may be, for example, set to 0.001 mass% or more and 2 mass% or less. The content is preferably 0.002 mass% or more and 1 mass% or less, preferably 0.005 mass% or more and 0.5 mass% or less, preferably 0.01 mass% or more and 0.2 mass% or less, and preferably 0.015 mass% or more and 0.1 mass% or less.

The topical composition of the present invention usually comprises water. The content of water in the composition of the present invention is, for example, 40 mass% or more, preferably 50 mass% or more, more preferably 60 mass% or more, still more preferably 70 mass% or more, even more preferably 75 mass% or more, particularly preferably 80 mass% or more, more particularly preferably 85 mass% or more, and most preferably 90 mass% or more. The upper limit of the content may be, for example, 95 mass%, 90 mass%, 85 mass%, or 80 mass%. The lower and upper limits mentioned above may be combined in any way. The topical composition of the present invention can effectively improve the stability of vitamin D3 even in cases in which the water content is increased and thus the issue of the stability of vitamin D3 becomes increasingly significant.

In cases in which the topical composition of the present invention comprises water, the pH of the topical composition of the present invention is preferably 6 to 8, more preferably 6.5 to 7.5, even more preferably 6.7 to 7.3, still more preferably 6.8 to 7.2, and particularly preferably 6.9 to 7.1, from the viewpoint of, for example, the stability of vitamin D3.

In one aspect of the present invention, the topical composition of the present invention can comprise components other than those described above. Examples of other components include hydrocarbons (such as liquid paraffin (e.g., light liquid paraffin) and squalane), solvents (such as benzyl alcohol, methyl ethyl ketone, N-alkylpyrrolidone, alkylene carbonate, acetone, ethanol, isopropanol, propylene glycol, 1,3-butylene glycol, concentrated glycerin, polyethylene glycol, hexylene glycol, and propylene carbonate), water-soluble polymers (such as carboxyvinyl polymer, hydroxymethylcellulose, hydroxyethylcellulose, hydroxypropylcellulose, hydroxypropyl methylcellulose, methylcellulose, chitosan, povidone, polyvinyl alcohol, gelatin, casein, carrageenan, sodium hyaluronate, 2-methacryloyloxyethyl phosphorylcholine-butyl methacrylate copolymer, xanthan gum, and polyethylene glycol), antioxidants, pH adjusters, preservatives, preservative agents, stabilizers, amino acids, chelating agents, and amphoteric surfactants, such as hydrogenated lecithin. These may be used alone or in any combination of two or more. Furthermore, the topical composition of the present invention may further comprise, as other components, active ingredients other than the vitamin D3 and glucocorticoid mentioned above, such as moisturizers, antiinflammatory agents, vitamin agents, antibacterial agents, sterilizers, antiviral agents, steroids, antibiotics, antipruritic agents, immunosuppressants, local anesthetics, and whitening agents.

In one aspect of the present invention, the topical composition of the present invention comprises hydrocarbons (hydrocarbon oil).

In one aspect of the present invention, the topical composition of the present invention comprises an antioxidant.

Examples of antioxidants include lipid-soluble antioxidants and water-soluble antioxidants. Examples of lipid-soluble antioxidants include butyl group-containing phenols, such as dibutylhydroxytoluene (BHT) and butylhydroxyanisole (BHA); nordihydroguaiaretic acid (NDGA); ascorbic acid esters, such as ascorbyl palmitate, ascorbyl stearate, aminopropyl ascorbyl phosphate, ascorbyl tocopherol phosphate, dl-α-tocopherol, d-δ-tocopherol, tocopherol acetate, ascorbyl triphosphate, and ascorbyl palmitate phosphate; gallic acid esters, such as ethyl gallate, propyl gallate, octyl gallate, and dodecyl gallate; propyl gallate; 3-butyl-4-hydroxyquinolin-2-one; carotenoids, such as lutein and astaxanthin; polyphenols, such as anthocyanins, catechin, tannin, and curcumin; and CoQ10. Examples of water-soluble antioxidants include ascorbic acid, ascorbic acid derivatives (such as ascorbic acid-2-sulfate disodium, sodium ascorbate, ascorbic acid-2-phosphate magnesium, and ascorbic acid-2-phosphate sodium), sodium hydrogen sulfite, sodium sulfite, sodium metabisulfite, sodium thiosulfate, and edetic acid or salts thereof (such as sodium edetate, disodium edetate, and tetrasodium edetate). The antioxidant is preferably a lipid-soluble antioxidant, more preferably a butyl group-containing phenol, and particularly preferably dibutylhydroxytoluene.

In cases in which the topical composition of the present invention comprises an antioxidant, the content of the antioxidant in the topical composition of the present invention is, for example, 0.001 to 5 mass%, preferably 0.01 to 2 mass%, and more preferably 0.05 to 0.5 mass%.

The content of other components in the topical composition of the present invention is not particularly limited as long as they do not significantly impair the effects of the present invention. The content is, for example, 0 mass% or more and 10 mass% or less, 0 mass% or more and 5 mass% or less, 0 mass% or more and 2 mass% or less, 0 mass% or more and 1 mass% or less, or 0 mass% or more and 0.1 mass% or less.

The topical composition of the present invention can be used for application to the skin. The topical composition of the present invention may be intended for application to the skin (excluding those intended for application to hair, such as shampoo and conditioner). The topical composition of the present invention is preferably in the form of a topical pharmaceutical or a topical composition, such as a cosmetic product. The topical composition of the present invention is intended for use without being removed after application, and is to be distinguished from cleansing agents that are designed to be removed (e.g., rinsed off) with water after application in foam form.

The drug form of the topical composition of the present invention is not particularly limited and any known form of a cosmetic product or quasi-drug may be used. Examples of such known forms include liquid agents, suspensions, emulsions, creams, gels, liniments, lotions, aerosols, powders, poultices, sheet agents obtained by impregnating a medicinal solution into sheets such as nonwoven fabrics, and emulsions for lip applications. Among these, liquid agents, suspensions, emulsions, creams, gels, and lotions are comfortable to use and thus preferred, and liquid agents (having transparency) are particularly preferred.

In one aspect of the present invention, the dosage form of the topical composition of the present invention is a gel, a liquid, an aerosol, a mist, or a foam.

The topical composition of the present invention can be prepared by mixing the components with a solvent. The composition of the present invention is preferably in a solubilized state. The "solubilized state" refers to a state in which surfactants form micelles, leading to transparent and uniform dissolution, thereby forming a single-phase solution. The solubilized state includes the state of a microemulsion. The microemulsion is formed from emulsified particles having a fine particle size of 100 nm or less, and is a thermodynamically stable micellar solution. The solubilized state can be confirmed by the transparent appearance. The solubilized state can also be confirmed by measuring the particle size according to conventional methods. The composition in the solubilized state can be obtained by dissolving each component under heating as necessary, followed by cooling.

It is preferable that the topical composition of the present invention is a composition that is loaded into a non-gas foam dispensing apparatus. The term "non-gas foam dispensing apparatus" used here refers to an apparatus with a mechanism that generates foam without relying on a propellant. Specifically, the topical composition of the present invention is preferably applied in the form of foam using a non-gas foam dispensing apparatus. Examples of the non-gas foam dispensing apparatus include a screen-type foam dispensing apparatus. The "screen-type foam dispensing apparatus" refers to an apparatus with a mechanism in which a liquid is pressurized and allowed to pass through a screen with a net-like structure (mesh), thereby enabling it to mix with air and generate foam. Examples of the screen-type foam dispensing apparatus include a pump-type foam dispensing apparatus, a tube-type foam dispensing apparatus, and a squeeze-type foam dispensing apparatus. Among these, a pump-type foam dispensing apparatus is particularly preferred.

The non-gas foam dispensing apparatus for use may be selected from, for example, known or commercially available apparatuses. For example, a pump-type foam dispensing apparatus, such as those disclosed in JP2012-45525A and JP2008-307478A, can be used.

The topical composition of the present invention can be used, for example, for the prevention or amelioration of psoriasis.

The term "amelioration" encompasses not only the attenuation or disappearance of symptoms, but also the suppression of the degree of worsening of symptoms or halting the worsening of symptoms in situations in which worsening of symptoms continues.

Psoriasis is not particularly limited and includes, for example, psoriasis vulgaris, psoriatic arthritis, guttate psoriasis, psoriatic erythroderma, and pustular psoriasis.

The application of the topical composition of the present invention to mammals, including humans, may be performed, for example, daily or once every several days, and is preferably performed continuously. When the topical composition of the present invention is applied continuously, the duration of application is preferably two weeks or more, more preferably three weeks or more, and still more preferably four weeks or more.

### Examples

The present invention is described below in detail based on Examples; however, the present invention is not limited to these Examples.

### Materials

The details of the materials used in the following test examples are as follows. "POE" denotes polyoxyethylene and "POP" denotes polyoxypropylene.

### a1. Drug substance: vitamin D3

- Calcipotriol monohydrate
- Maxacalcitol

### a2. Drug substance: glucocorticoid

- Betamethasone dipropionate
- Betamethasone butyrate propionate

### b. Nonionic surfactant A

- Polyoxyethylene hydrogenated castor oil 40 (HLB value: 12.5); "40" indicates the average number of moles of ethylene oxide added.

### c1. Nonionic surfactant B: polyoxyalkylene alkyl ether

- POE(20)POP(4) cetyl ether (HLB value: 12.5)
- POE lauryl ether (HLB value: 14.5)

### c2. Nonionic surfactant B: polyoxyalkylene glycol

- POEPOP glycol (HLB value: 10.9)

### c3. Nonionic surfactant B: polyoxyalkylene fatty acid ester

- Polyethylene glycol monostearate (HLB value: 17.5)

### c4. Nonionic surfactant B: polyoxyalkylene sorbitol fatty acid ester

- Polyoxyethylene sorbitol tetraoleate (HLB value: 15.5)

### c5. Nonionic surfactant B: polyglycerol fatty acid ester

- Polyglyceryl monolaurate (HLB value: 15.5)

### d. Ester oil

- Diisopropyl adipate
- Glyceryl triisooctanoate

### e. pH Adjuster

- Sodium hydroxide
- Sodium citrate hydrate
- Citric acid hydrate

### Test Example 1: Evaluation Test 1 for Stability of Drug Substance

Liquid compositions (Examples 1 to 9 and Comparative Examples 1 to 11) were prepared in accordance with the formulations shown in Table 1 and Table 2 (in the tables, the numerical values are expressed in mass% and blank cells indicate 0). Specifically, the group a above (a1 and a2) and the group d1 were dissolved, and the group b and the group c (c1, c2, c3, c4, or c5) were then added and dissolved again. Subsequently, water was added and stirred for dissolution to thereby obtain liquid compositions. As shown in Table 1 and Table 2, the pH of the liquid compositions was adjusted to 7.0 by the addition of the pH adjusting agent (in an appropriate amount of 0.002 to 0.01 mass%, as shown in the tables).

The stability of vitamin D3 and glucocorticoid in the obtained liquid compositions was measured. Specifically, the liquid compositions were left to stand at 60°C, and at 1 week, 2 weeks, and 3 weeks, the amounts of vitamin D3 and glucocorticoid present in each liquid composition were analyzed by HPLC, and the peak areas of the samples were measured using automatic integration to calculate the residual rates of vitamin D3 and glucocorticoid.

**Table 1**

| | | Examples | | | | | | | | |
|---|---|---|---|---|---|---|---|---|---|---|
| | | 1 | 2 | 3 | 4 | 5 | 6 | 7 | 8 | 9 |
| a1 | Calcipotriol monohydrate | 0.0052 | 0.0052 | 0.0052 | 0.0052 | 0.0052 | 0.0052 | | | |
| | Maxacalcitol | | | | | | | 0.0025 | 0.0025 | 0.0025 |
| a2 | Betamethasone dipropionate | 0.06 | 0.06 | 0.06 | 0.06 | 0.06 | 0.06 | | | |
| | Betamethasone butyrate propionate | | | | | | | 0.05 | 0.05 | 0.05 |
| b | POE hydrogenated castor oil 40 | 4.00 | 2.50 | 4.36 | 4.95 | 4.17 | 4.17 | 4.00 | 2.50 | 4.95 |
| c1 | POE(20)POP(4) cetyl ether | 1.00 | | | | | | 1.00 | | |
| | POE lauryl ether | | 2.50 | | | | | | 2.50 | |
| c2 | POEPOP glycol | | | 0.64 | | | | | | |
| c3 | Polyethylene glycol monostearate | | | | 0.05 | | | | | 0.05 |
| c4 | Polyoxyethylene sorbitol tetraoleate | | | | | 0.83 | | | | |
| c5 | Polyglyceryl monolaurate | | | | | | 0.83 | | | |
| d | Diisopropyl adipate | 1.3 | 1.3 | 1.3 | 1.3 | 1.3 | 1.3 | 1.3 | 1.3 | 1.3 |
| | Glyceryl triisooctanoate | 0.2 | 0.2 | 0.2 | 0.2 | 0.2 | 0.2 | 0.2 | 0.2 | 0.2 |
| e | Sodium hydroxide | q.s. | q.s. | q.s. | q.s. | q.s. | q.s. | q.s. | q.s. | q.s. |
| | Sodium citrate hydrate | 0.6 | 0.6 | 0.6 | 0.6 | 0.6 | 0.6 | 0.6 | 0.6 | 0.6 |
| | Citric acid hydrate | q.s. | q.s. | q.s. | q.s. | q.s. | q.s. | q.s. | q.s. | q.s. |
| Water | | Balance | Balance | Balance | Balance | Balance | Balance | Balance | Balance | Balance |

**Table 2**

| | | Comparative Examples | | | | | | | | | | |
|---|---|---|---|---|---|---|---|---|---|---|---|---|
| | | 1 | 2 | 3 | 4 | 5 | 6 | 7 | 8 | 9 | 10 | 11 |
| a1 | Calcipotriol monohydrate | 0.0052 | 0.0052 | 0.0052 | 0.0052 | 0.0052 | 0.0052 | 0.0052 | | | | |
| | Maxacalcitol | | | | | | | | 0.0025 | 0.0025 | 0.0025 | 0.0025 |
| a2 | Betamethasone dipropionate | 0.06 | 0.06 | 0.06 | 0.06 | 0.06 | 0.06 | 0.06 | | | | |
| | Betamethasone butyrate propionate | | | | | | | | 0.05 | 0.05 | 0.05 | 0.05 |
| b | POE hydrogenated castor oil 40 | 5.00 | | | | | | | 5.00 | | | |
| c1 | POE(20)POP(4) cetyl ether | | 5.00 | | | | | | | 5.00 | | |
| | POE lauryl ether | | | 5.00 | | | | | | | 5.00 | |
| c2 | POEPOP glycol | | | | 5.00 | | | | | | | |
| c3 | Polyethylene glycol monostearate | | | | | 5.00 | | | | | | 5.00 |
| c4 | Polyoxyethylene sorbitol tetraoleate | | | | | | 5.00 | | | | | |
| c5 | Polyglyceryl monolaurate | | | | | | | 5.00 | | | | |
| d | Diisopropyl adipate | 1.3 | 1.3 | 1.3 | 1.3 | 1.3 | 1.3 | 1.3 | 1.3 | 1.3 | 1.3 | 1.3 |
| | Glyceryl triisooctanoate | 0.2 | 0.2 | 0.2 | 0.2 | 0.2 | 0.2 | 0.2 | 0.2 | 0.2 | 0.2 | 0.2 |
| e | Sodium hydroxide | q.s. | q.s. | q.s. | q.s. | q.s. | q.s. | q.s. | q.s. | q.s. | q.s. | q.s. |
| | Sodium citrate hydrate | 0.6 | 0.6 | 0.6 | 0.6 | 0.6 | 0.6 | 0.6 | 0.6 | 0.6 | 0.6 | 0.6 |
| | Citric acid hydrate | q.s. | q.s. | q.s. | q.s. | q.s. | q.s. | q.s. | q.s. | q.s. | q.s. | q.s. |
| Water | | Balance | Balance | Balance | Balance | Balance | Balance | Balance | Balance | Balance | Balance | Balance |

Figs. 1 to 9 show the results. The results revealed that the use of the nonionic surfactant A, which is a polyoxyalkylene hydrogenated castor oil, and the nonionic surfactant B, which is a nonionic surfactant other than polyoxyalkylene hydrogenated castor oils, in combination produced a synergistic effect with respect to improving the stability of vitamin D3. The results also revealed that this combination does not adversely affect the stability of glucocorticoid.

### Test Example 2: Evaluation Test 2 for Stability of Drug Substance

Liquid compositions were prepared in the same manner as in Examples 3 and 6 of the present application, except that an antioxidant (dibutylhydroxytoluene) was added to a concentration of 0.1 mass% (Examples 3A and 6A). The stability of vitamin D3 in the obtained liquid compositions was measured according to the method described in Test Example 1.

Figs. 10 and 11 show the results. The results revealed that the antioxidant does not adversely affect the stability of vitamin D3, but rather enhances the stability of vitamin D3.

## Claims

1. A topical composition comprising vitamin D3, a nonionic surfactant A, which is a polyoxyalkylene hydrogenated castor oil having an average number of moles of alkylene oxide added of 55 or less, and a nonionic surfactant B, which is a nonionic surfactant other than polyoxyalkylene hydrogenated castor oils.

2. The topical composition according to claim 1, wherein the nonionic surfactant B is at least one selected from the group consisting of polyoxyalkylene alkyl ethers, polyoxyalkylene glycols, polyoxyalkylene fatty acid esters, polyoxyalkylene sorbitol fatty acid esters, and polyglycerol fatty acid esters.

3. The topical composition according to claim 1, wherein the nonionic surfactant B has an HLB value of 6 or more.

4. The topical composition according to claim 1, wherein the total content of the nonionic surfactant A and the nonionic surfactant B in the topical composition is 0.5 mass% or more and 10 mass% or less.

5. The topical composition according to claim 1, wherein the content of the nonionic surfactant A in the total of the nonionic surfactant A and the nonionic surfactant B is 10 mass% or more and 99.9 mass% or less.

6. The topical composition according to claim 1, which comprises an ester oil.

7. The topical composition according to claim 6, wherein the ester oil comprises at least glyceryl triisooctanoate.

8. The topical composition according to claim 1, wherein
the nonionic surfactant B is at least one selected from the group consisting of polyoxyalkylene alkyl ethers, polyoxyalkylene glycols, polyoxyalkylene fatty acid esters, polyoxyalkylene sorbitol fatty acid esters, and polyglycerol fatty acid esters,
the nonionic surfactant B has an HLB value of 6 or more,
the total content of the nonionic surfactant A and the nonionic surfactant B in the topical composition is 1 mass% or more and 10 mass% or less,
the content of the nonionic surfactant A in the total of the nonionic surfactant A and the nonionic surfactant B is 10 mass% or more and 99.9 mass% or less, and
the topical composition comprises an ester oil.

9. The topical composition according to claim 1, wherein the vitamin D3 is at least one selected from the group consisting of calcipotriol, maxacalcitol, alfacalcidol, falecalcitriol, tacalcitol, and calcitriol.

10. The topical composition according to any one of claims 1 to 9, wherein the nonionic surfactant B is at least one selected from polyoxyethylene polyoxypropylene cetyl ether, polyoxyethylene lauryl ether, polyoxyethylene polyoxypropylene glycol, polyethylene glycol monostearate, polyoxyethylene sorbitol tetraoleate, and polyglyceryl monolaurate.

11. The topical composition according to any one of claims 1 to 10, which further comprises water.

12. The topical composition according to claim 11, wherein the content of water in the topical composition is 40 mass% or more.

13. The topical composition according to claim 11, wherein the content of water in the topical composition is 75 mass% or more.

14. The topical composition according to any one of claims 1 to 13, wherein the content of the vitamin D3 in the topical composition is 0.0001 mass% or more and 0.2 mass% or less.

15. The topical composition according to any one of claims 1 to 14, which further comprises an antioxidant.

16. The topical composition according to any one of claims 1 to 15, wherein the dosage form is a gel, liquid, aerosol, mist, or foam.

17. The topical composition according to any one of claims 1 to 16 for use in the prevention or amelioration of psoriasis.
